Europäisches Patentamt

(19) European Patent Office

Office européen des brevets

(11) Publication number : **0 359 807 B1**

(12) **EUROPEAN PATENT SPECIFICATION**

(45) Date of publication of patent specification :
**17.11.94 Bulletin 94/46**

(51) Int. Cl.⁵ : **G01N 33/58,** G01N 33/552,
G01N 21/64

(21) Application number : **89904182.6**

(22) Date of filing : **28.03.89**

(86) International application number :
**PCT/GB89/00320**

(87) International publication number :
**WO 89/09408 05.10.89 Gazette 89/24**

(54) **METHOD OF ASSAY.**

(30) Priority : **29.03.88 GB 8807488**

(43) Date of publication of application :
**28.03.90 Bulletin 90/13**

(45) Publication of the grant of the patent :
**17.11.94 Bulletin 94/46**

(84) Designated Contracting States :
**AT BE CH DE FR GB IT LI LU NL SE**

(56) References cited :
**EP-A- 0 075 353
EP-A- 0 103 426
EP-A- 0 211 587
EP-A- 0 214 768
EP-A- 0 242 527
Proc.Electrochem.Soc.,volume 87-
9,(Proc.Symp.Chem.Sens.),1987,A.L.Har-
mer:"Guided-wave chemical sensors",pages
409-427 see page 416,line 32 - page 419,last
line**

(73) Proprietor : **ARS Holding 89 N.V.
6 John B. Gorsiraweg
Curaçao (AN)**

(72) Inventor : **FLANAGAN, Michael, Thomas
17 Chinnery Hill
Bishop's Stortford
Hertfordshire CM23 3PS (GB)**
Inventor : **ASHWORTH, Robert, Heddle
18 Downshire Hill
Hampstead London NW3 1NT (GB)**

(74) Representative : **Woodman, Derek et al
Frank B. Dehn & Co.
European Patent Attorneys
Imperial House
15-19 Kingsway
London WC2B 6UZ (GB)**

## Description

This invention relates to assay techniques and to means for putting such techniques into effect. In particular it relates to an improved assay technique providing an enhanced signal-to-noise ratio and enhanced sensitivity.

Fluorescence assays, for example fluorescence immunoassays, where one component of the assay is immobilised on a waveguide interface, give rise to the problem of differentiating between fluorescence emission from a labelled further assay reagent that is bound to the surface (the required signal) and fluorescence emission from that further reagent which is still in solution giving unwanted noise signal.

A greater differentiation between these two populations of bound and unbound reagent would improve the signal-to-noise ratio and sensitivity of the assay.

The invention described below makes use of the evanescent field properties of optical waveguides combined with a resonance energy transfer step [Forster Energy Transfer] between a fluorophore bound to a reagent X, on or near an immobilised detection reagent Y, for example an antibody, and in some cases, dyes immobilised on the waveguide, or within an immobilised film support.

The differentiation between bound and free reagents in homogeneous assays of this type is achieved because energy transfer will only occur efficiently across molecular dimensions and hence will be observed only on complex formation.

Known evanescent waveguide assays are based on the assumption that the penetration of the evanescent wave is negligible beyond molecular dimensions and hence only excites the label on the complex. However, in reality, this assumption is an oversimplification and the sensitivity of such assays is less than might be expected.

EP-A-211 587 discloses a dielectric waveguide sensor for use in spectrophotometric assays of waveguides in fluids. This waveguide has a core with a refractive index which is greater than the refractive index of the fluid. A dopant is located within the core which can be excited by a primary signal radiation emitted by the analyte so as to emit a secondary signal radiation, which is detected by monitoring the waveguide core. The waveguide is aligned so that bound analyte is excited by an evanescent wave arising from modes of electromagnetic radiation propagated down the waveguide core.

EP-A-242527 describes a method for detecting the presence of an analyte which involves forming a complex comprising the analyte and a binding entity. The binding entity comprises a recognition segment and a first partner of an energy transfer system. The complex is thereafter contacted with a reporting entity to form a unit, said reporting entity comprising a second partner of the energy transfer system. The unit is directly illuminated with energy which can be absorbed only by one of said partners. This partner emits fluorescent energy some of which is absorbed by the other of said partners. This also fluoresces, but at longer wavelength than the first partner and possibly also for a longer duration, allowing the presence of the analyte to be verified.

According to one aspect of the present invention there is provided a method of assay of a ligand in a sample which comprises

(A) incubating, simultaneously or in any desired sequence,
    (a) the sample,
    (b) a reagent X labelled with a first energy acceptor,
    (c) a reagent Y immobilised directly or indirectly on the surface of an optical waveguide,
    (d) a second energy acceptor immobilised directly or indirectly (if desired via reagent Y) on the surface of the optical waveguide,
    (e) if desired, at least one further energy acceptor, which may be the same as or different from said first or second energy acceptors, immobilised directly or indirectly on or within the surface of said optical waveguide, wherein one of the reagents X and Y comprises a specific binding partner to said ligand and the other reagents X and Y comprises either a ligand analogue or a specific binding partner of said ligand and wherein one of the said first and second energy acceptors comprises a fluorophore (a "donor fluorophore") the electronic emission spectrum of which overlaps with the electronic absorption spectrum of the other of said first and second energy acceptors;

(B) irradiating the said optical waveguide with electromagnetic radiation of a wavelength suitable for exciting said donor fluorophore; and

(C) detecting whether and, if desired, the extend to which and/or rate at which, resonance energy transfer occurs, either directly or indirectly, between the first and second energy acceptors or between the second and first energy acceptors respectively: wherein either the irradiation of step (B) is by means of the evanescent field produced by electromagnetic radiation propagating along said waveguide and/or the detection step (C) utilises evanescent field coupling into the waveguide of the fluorescence of the first energy acceptor or the fluorescence, if any, of the second energy acceptor.

The term "ligand analogue" as used herein refers to a species capable of complexing with the same binding site of the same specific binding partner as the ligand under assay, and includes inter alia within its scope a known quantity of the ligand under assay.

The term "energy acceptor" as used herein denotes a compound which is capable of absorbing electromagnetic energy, for example, a dye or a fluorophore. Typical energy acceptors include, for example, those of the coumarin, fluorescein, lucifer yellow and rhodamine families, phycobiliproteins and erythrosin: specific examples of energy acceptors include fluorescein isothiocyanate, rhodamine B, rhodamine 6G, rhodamine 123, R-phycoerythrin, c-phycocyanin, allophycocyanin, fluorescamine, lucifer yellow VS and lucifer yellow CH.

As mentioned above, one of the said first and second energy acceptors is a donor fluorophore. The other of the said first and second energy acceptors may be an acceptor fluorophore or an acceptor dye. The energy acceptors are chosen such that excitation of the donor fluorophore by electromagnetic radiation of a suitable wavelength and formation of a direct or indirect complex between reagents X and Y facilitates resonance energy transfer, either directly or indirectly, between the energy acceptors. Thus, when the first and second energy acceptors comprise a donor fluorophore and an acceptor dye or an acceptor dye and a donor . fluorophore respectively, the fluorescence of the donor fluorophore is quenched and when the first and second energy acceptors comprise a donor fluorophore and an acceptor fluorophore or an acceptor fluorophore and a donor fluorophore respectively, the fluorescence of the donor fluorophore is quenched and the acceptor fluorophore fluoresces (although the fluorescence of the acceptor fluorophore may have a very low quantum efficiency).

By way of example only, representative pairs of compounds which are suitable for use as donor and acceptor fluorophores in the invention are listed in Table 1 below.

## Table 1

| Donor fluorophore | Corresponding acceptor fluorophore |
|---|---|
| fluorescein isothiocyanate | rhodamine B |
| R-phycoerythrin | allophycocyanin |
| fluorescein isothiocyanate | R-phycoerythrin |
| rhodamine B | allophycocyanin |
| fluorescamine | lucifer yellow VS |
| c-phycocyanin | R-phycoerythrin |

When at least one further energy acceptor is present as component (e) two or more resonance energy transfer steps may occur. Thus, for example, when the first energy acceptor is an acceptor dye, the second energy acceptor is a donor fluorophore and an acceptor fluorophore is present as component (e), complex formation between reagents X and Y facilitates resonance energy transfer between the donor fluorophore and the acceptor dye such that the acceptor dye quenches the fluorescence emitted by both the donor fluorophore and the acceptor fluorophore present as component (e).

By detecting whether resonance energy transfer between the first and second energy acceptors or vice versa occurs, it may be possible to detect the quenching of the fluorescence of the donor fluorophore and/or, when present, the fluorescence of an acceptor fluorophore. When one or more further energy acceptors are present as component (e) the quenching and/or fluorescence of one or more components may be detected. Thus, for example, when the first energy acceptor is an acceptor dye, the second energy acceptor is a donor fluorophore and an acceptor fluorophore is present as component (e) quenching of the fluorescence of the donor fluorophore and/or the acceptor fluorophore may be detected.

Where one of reagents X and Y comprises a ligand analogue and the other of reagents X and Y comprises a specific binding partner to the ligand, complex formation between reagents X and Y may occur directly. Alternatively, where both reagents X and Y comprise specific binding partners to the ligand, complex formation between reagents X and Y indirectly via the ligand, if present in the sample, may occur.

It is to be understood that reagent Y may be immobilised on the surface of the optical waveguide either directly or indirectly. For example, when reagent Y is an antibody, indirect immobilisation may be effected by means of an anti-species antibody to reagent Y which is itself bound to the surface of the optical waveguide.

Immobilization on the surface of the optical waveguide may thus occur by means of immunological bonding as in an antibody-antigen or antibody-hapten bond or non-immunological bonding such as that between a protein and a ligand, e.g. between avidin and biotin.

Suitable materials for the optical waveguide include, for example, glass (e.g. permabloc glass or crown glass), plastics (e.g. acrylic or polystyrene), silica and quartz; but in principle any material of suitable refractive index may be used which is transparent to radiation at least at the wavelengths involved in the assay.

Preferably both the irradiation step (B) and the detection step (C) utilise evanescent-field coupling.

Several advantages arise by combining the two techniques of resonance energy transfer and evanescent-field coupling.

1. The convolution of the inverse sixth power dependence on distance of resonance transfer with the exponential decay of the evanescent wave restricts the localization of the excitation region closer to the waveguide.

2. The Stokes shift in frequency between the exciting electromagnetic radiation and the emitted light is increased by a suitable choice of donor fluorophore and, if present, the acceptor fluorophore, thus allowing more effective filtering of the signal.

3. The signal-to-noise ratio is improved. When an acceptor fluorophore is present the measurement of the acceptor fluorescence signal in the presence of donor background is facilitated by immobilization of the acceptor on the waveguide surface. The number of transfer pathways can be increased by immobilizing further donors or acceptors on or within the waveguide surface.

4. The Stokes shift can be further increased by the coimmobilization of additional fluorophore species, emitting at longer wavelengths, which will allow a chain of transfer steps to occur. Such a chain of transfer steps and an increased number of transfer pathways cannot be effectively incorporated within a conventional homogeneous assay.

5. Transfer along a chain of fluorophores will be more efficient if the immobilised fluorophores are appropriately aligned. Such alignments can be achieved with liquid crystal and Langmuir-Blodgett film deposition in combination with the fluorophore immobilization.

The optical waveguide mentioned hereinbefore may optionally be incorporated in a grating arrangement such as is described in our copending application of even date, published as EP-A-0363467 and entitled "Waveguide Sensor". Such a grating arrangement, used in combination with the above transfer techniques, will allow the separation and filtering of the emitted and exciting radiation within the waveguiding sensor device itself, removing the need for external monochromators or filters.

It is especially to be noted that devices of the type described and claimed in EP-A-171148 may be advantageously adapted to carry the test samples to be assayed in the method of the present invention.

The emitted radiation may be filtered and/or collimated, if desired, before being detected by conventional means, for example one or more photomultiplier tubes.

According to another aspect of the invention there is provided a kit for carrying out a method of assay as described herein which comprises (i) a reagent X as defined above and (ii) an optical waveguide on the surface of which is immobilised (directly or indirectly) a reagent Y as defined above and on which optical waveguide is immobilised (directly or indirectly, if desired via reagent Y) at least one further energy acceptor as defined above.

The invention will be particularly described hereinafter with reference to an antigen as the ligand and where reagents X and Y each comprise an antibody i.e. a sandwich-type immunoassay. However, the invention is also applicable to competition-type immunoassays, for example where one of reagents X and Y comprises an antibody and the other comprises a ligand analogue.

Furthermore, the invention is not to be taken as limited to assays of antibodies or antigens. Examples of ligands which may be assayed by the method of the invention are given in Table 2 below, together with an indication of a suitable specific binding partner in each instance.

## Table 2

| Ligand | Specific Binding Partner |
| --- | --- |
| antigen | specific antibody |
| antibody | antigen |
| hormone | hormone receptor |
| hormone receptor | hormone |
| polynucleotide strand | complementary polynucleotide strand |
| avidin | biotin |
| biotin | avidin |
| protein A | immunoglobulin |
| immunoglobulin | protein A |
| enzyme | enzyme cofactor (substrate) or inhibitor |
| enzyme cofactor (substrate) or inhibitor | enzyme |
| lectins | specific carbohydrate |
| specific carbohydrate of lectins | lectins |

The method of the invention has very broad applicability but in particular may be used to assay: hormones, including peptide hormones (e.g. thyroid stimulating hormone (TSH), luteinising hormone (LH), human chorionic gonadotrophin (hCG), follicle stimulating hormone (FSH), insulin and prolactin) or non-peptide hormones (e.g. steroid hormones such as cortisol, estradiol, progesterone and testosterone, or thyroid hormones such as thyroxine (T4) and triiodothyronine), proteins (e.g. carcinoembryonic antigen (CEA) and alphafetoprotein (AFP)), drugs (e.g. digoxin), sugars, toxins, vitamins, viruses such as influenza, para-influenza, adeno-, hepatitis, respiratory and AIDS viruses, or microorganisms.

It will be understood that the term "antibody" used herein includes within its scope:

(a) any of the various classes or sub-classes of immunoglobulin, e.g. IgG, IgM, derived from any of the animals conventionally used, e.g. sheep, rabbits, goats or mice,

(b) monoclonal antibodies,

(c) intact molecules or "fragments" of antibodies, monoclonal or polyclonal, the fragments being those which contain the binding region of the antibody, i.e. fragments devoid of the Fc portion (e.g. Fab, Fab', $F(ab')_2$) or the so-called "half-molecule" fragments obtained by reductive cleavage of the disulphide bonds connecting the heavy chain components in the intact antibody. The method of preparation of fragments of antibodies is well known in the art and will not be described herein.

The term "antigen" as used herein will be understood to include both permanently antigenic species (for example, proteins, bacteria, bacterial fragments, cells, cell fragments and viruses) and haptens which may be rendered antigenic under suitable conditions.

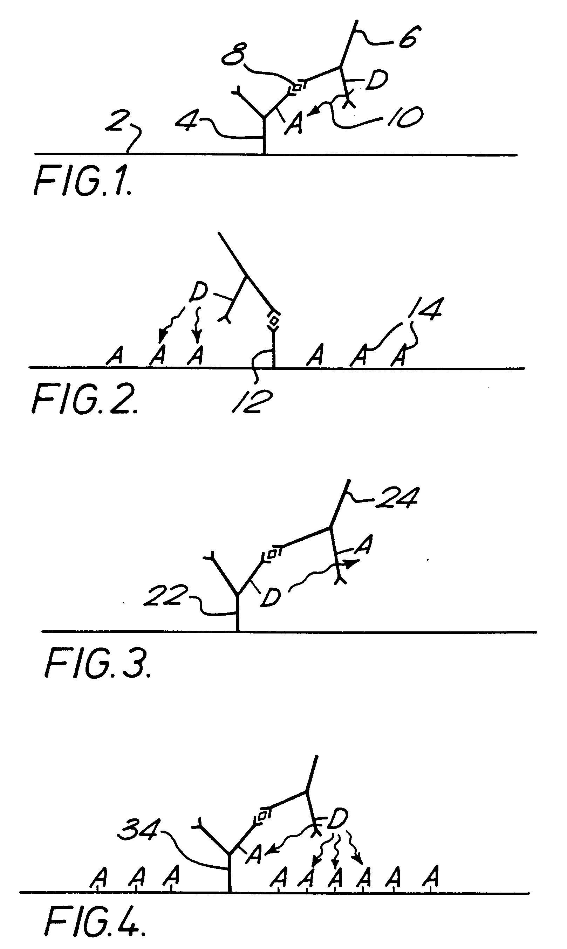

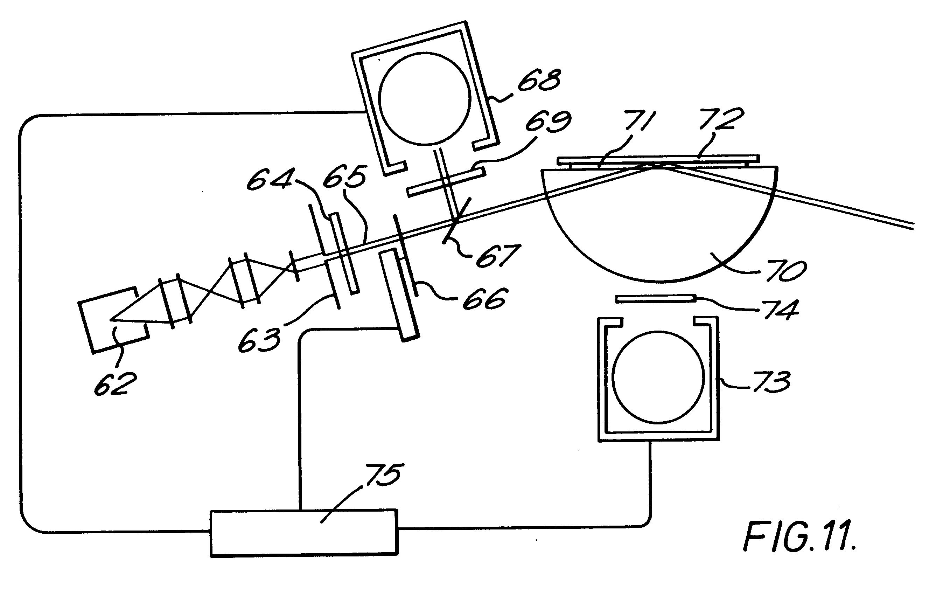

For a better understanding of the present invention, reference is made to the accompanying drawings wherein Figures 1 to 10 depict schematically various assay formats according to the invention and Figure 11 shows schematically an arrangement of apparatus which is suitable for carrying out the method of the invention.

In the embodiments depicted in the drawings Forster energy transfer occurs from an excited "donor" fluorophore (D) to an "acceptor" fluorophore (A). The donor fluorophore is excited either by an evanescent field of light guided within the waveguide to which the assay complex is immobilised or directly by light from above or below the waveguide.

Fluorescent emission is collected from both, or either, of the donor and acceptor fluorophores. The fluorescent emission is either coupled into waveguide modes or (when the donor fluorophore is excited by an evanescent field of light) may be transmitted across the waveguide prior to detection.

Fig. 1 shows the surface 2 of an optical waveguide with an antibody 4 (reagent Y) immobilised thereon, which antibody is labelled with an acceptor fluorophore (A). A second antibody 6, labelled with a donor fluorophore (D) is present as reagent X.

During the assay, an antigen 8 (i.e. the ligand) in the sample becomes bound to binding sites both on the A-labelled antibody 4 and on the D-labelled antibody 6, thereby forming a "sandwich complex". The donor fluorophore D is electronically excited by absorption of incident radiation. Forster energy transfer 10 then occurs between the donor fluorophore D and the acceptor fluorophore A, causing the latter to become electronically excited, whilst the donor fluorophore is deactivated and so loses its ability to fluoresce. During the assay, the fluorescence from either or both fluorophores may be monitored.

In a further embodiment of the invention shown in Fig. 2 the immobilised reagent Y is the Fab fragment 12 of an antibody. The use of such fragments reduces the distance between the donor and the acceptor fluorophores and hence makes energy transfer more efficient.

In this embodiment, acceptor fluorophores 14 are immobilised on the surface of the optical waveguide.

Fig. 3 shows the reverse of the situation shown in Fig. 1. The immobilised antibody 22 (reagent Y) is labelled with a donor fluorophore (D) and reagent X comprises a second antibody 24 labelled with an acceptor fluorophore (A).

If the acceptor fluorophore has a very low quantum yield of fluorescence it may be preferable to detect the quenching of the donor fluorescent emission intensity rather than detecting an increase from zero intensity of the emission from the fluorescent acceptor. An acceptor dye provides a limiting case of an acceptor fluorophore the fluorescence quantum yield of which is negligibly small

Fig. 4 shows a waveguide surface on which are immobilised an acceptor fluorophore (A) and an antibody 34 labelled with the same type of acceptor fluorophore (A). This arrangement increases the efficiency of energy transfer from the donor fluorophore (D) by reducing the distance between donor and acceptor and increasing the number of transfer pathways.

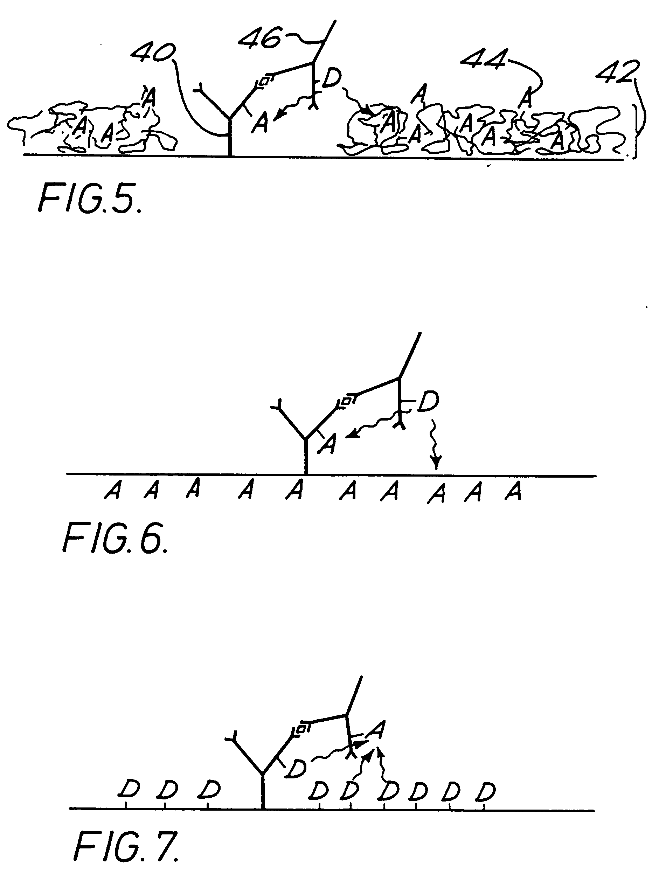

In Fig. 5, in addition to an immobilised antibody 40 labelled with acceptor fluorophore and the immobilised acceptor fluorophore as in Fig. 4, a polymer layer 42 is also immobilised onto the waveguide surface. This polymer layer may also be labelled with an acceptor fluorophore (A) 44 and this serves to reduce the average distance between the donor and acceptor fluorophores. An appropriate choice of polymer will also reduce the amount of non-specific binding of the donor fluorophore (D) - labelled antibody 46 to the waveguide surface, which would give unwanted noise signal.

Alternatively, the acceptor fluorophore may, as shown in Fig. 6, be absorbed within the waveguide surface as well as being used to label the immobilised antibody. This avoids the loss of fluorescence emission on transmission through the upper waveguide interface.

Fig. 7 shows an embodiment similar to that of Fig. 4 except that the positions of the donor and acceptor fluorophores are reversed.

Fig. 8 shows an immobilised molecular matrix which both incorporates acceptor fluorophores and aligns them. This serves to change the angular profile of acceptor fluorescence emission in order to improve the efficiency with which it is collected by the collimation and detection system. If the donor and acceptor positions are exchanged then efficient excitation of the donor can be arranged as shown in Fig. 9.

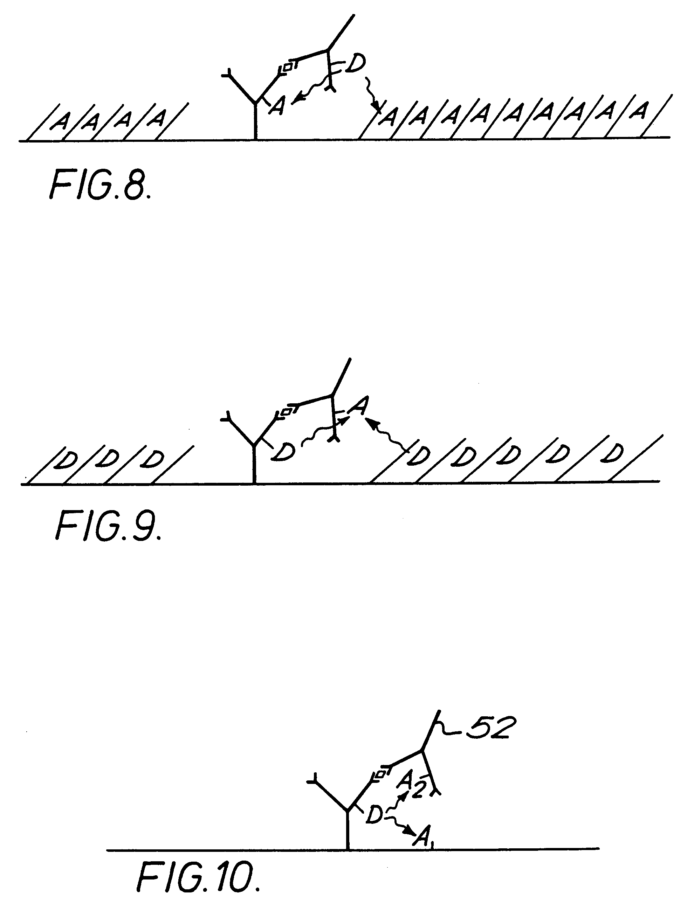

In a further embodiment of the invention, shown in Fig. 10, the binding of an antibody 52 labelled with an energy acceptor which does not fluoresce e.g. a dye (A2) will reduce the transfer of energy from a donor fluorophore (D) to an acceptor fluorophore (A1). This combines the detection of quenching of A1 with an improved Stokes shift introduced by the energy transfer step.

In each of the embodiments depicted in Figs. 4 to 10 an enhanced Stokes shift separation between exciting and emission wavelengths can be achieved by coimmobilization of more than one donor or acceptor species at the waveguide surface, on the polymer, or within the aligning matrix. The aligning matrix may itself be fluorescent. The enhancement results from the successive transfer from one fluorescent species to the next.

Fig. 11 shows an experimental set-up suitable for carrying out an assay in accordance with the method of the invention. Radiation of a wavelength suitable for the assay emerges from a light source 62 and, after collimation by a conventional system of lenses, passes through a slit 63 and a filter 64. The resulting beam of radiation 65 is chopped by a chopper 66 and then split by means of a beam splitter 67: the reflected part of the beam (the reference beam) passes into a photomultiplier tube 68, via a neutral density filter 69; the transmitted part of the beam passes through a hemicylindrical prism 70 and into a waveguide 71 which is attached to the prism by means of a transparent index-matching fluid or glue. In this example, sample liquid is drawn into a capillary cavity between the waveguide 71 and an upper plate 72, as would be found, for example, in a capillary fill device (see, for example, EP-A-171148). Light which emerges from the prism 70 is detected by one or more than one photomultiplier tube 73 after passing through one or more than one suitable filter 74. The photomulitplier tubes 68 and 73 and the chopper 66 are integrated by means of a lock-in amplifier 75. The signals from the photomultiplier tubes may be processed and output as desired.

The following non-limiting Example illustrates the present invention.

### Example 1

### Assay of Human Chorionic Gonadotrophin (hCG)

In this assay, fluorescently labelled antibody becomes bound as a result of formation of a sandwich complex with the analyte ligand (hCG) and a second antibody, also labelled with fluorophore, which is linked to the waveguide.

### Preparation of Starting Materials

### (i) Fabrication of avidin-coated waveguides

Glass waveguides, 10 x 20 x 1.1 mm, were cut out of Permabloc (trade mark) glass (Pilkington Glass Ltd., St. Helens, UK) using standard glass scribing techniques. After thorough washing with detergent and ultrasonic agitation, the glass was activated with a silane (8% 3-glycidoxypropyl-trimethoxysilane (Fluka, Glossop, UK)) at pH 3.5 for 2 hours. The glass was then washed and an appropriate cross-linking agent (e.g. SMCC, succinimidyl 4-(N-maleimidomethyl)cyclohexane-1-carboxylate (Pierce, Luton, England) or glutaraldehyde) was used to couple avidin to the glass surface using standard techniques (see, for example, Ishikura et al, Journal of Immunoassay 4, 209-237(1983)). The glass slides were then spin-coated with 10% sucrose solution containing 0.1% casein and stored dry at 4°C until use.

### (ii) Preparation of biotin/R-phycoerythrin/anti-hCG antibody

Monoclonal anti-hCG antibodies were obtained from mouse ascites fluid by the method of Milstein and Kohler in Nature 256, 495-497 (1975). Antibodies from individual hybridoma cell lines were screened to identify those producing antibody to discrete antigen determinants. Those having the highest affinities to hCG were selected for use in the assay. 1.48 mg R-phycoerythrin was mixed with 0.34 mg N-hydroxy-succinimido-biotin ester (Sigma, Poole, UK) using standard methods (see Guesdon et al, J. Histochem. Cytochem. 27, 1131 (1979)). The resulting phycoerythrin/biotin conjugate was purified using a Pharmacia PD10 column employing 0.2 M sodium bicarbonate buffer, pH 9.0 then activated with SMCC (see Ishikura et al, Journal of Immunoassay 4 209-327 (1983)). Antibody A was activated with 2-iminothiolane and then mixed with the phycoerythrin/biotin conjugate in equal amounts and left overnight at 4°C. The conjugate is purified on a TSK 3000 SWG (trade mark) column eluted with a triethanolamine buffer (0.1 M, pH 7.3) containing NaCl (0.1 M), $MgCl_2$ ($10^{-3}$M), $ZnCl_2$ ($10^{-3}$M) and 1% $NaN_3$, the fractions of highest-molecular weight being collected.

### (iii) Preparation of allophycocyanin/anti-hCG antibody

Antibody B (a second monoclonal antibody to hCG, specific for a different antigenic determinant) was conjugated to allophycocyanin following methods outlined in Stryer et al, US 4,542,104(1985).

### (iv) Preparation of hCG standard solutions

A freeze-dried preparation of hCG, calibrated against the first international reference preparation (75/537) was obtained from Biodata SpA, Milan, Italy. This sample was diluted in horse serum (Serono Diagnostics, Woking, England) to the desired concentration.

### Apparatus for optical measurements

An optical apparatus suitable for conducting an assay according to the present invention is shown in figure 11 of the accompanying drawings. The light source 62 is a 150 W xenon arc lamp, the excitation filter 64 has a centre wavelength of 485 nm (60 nm bandwidth), the detection filter has a centre wavelength of 580 nm (70 nm bandwidth). These filters are suitable for an assay involving R-phycoerythrin. A long-pass collector for allophycocyanin had a cut-on at 645 nm. A Hakuto R928 photomultiplier tube (Hakuto, Waltham Cross UK) is employed as the photodetectors 68 & 73 and an EG and G 5207 (PAR, Precton, USA) lock-in amplifier 75 is employed. The glass waveguide 71 may be mounted in a cell of sufficiently small dimensions so as to allow the sample to be drawn over the waveguide surface via capillary action.

Assay procedure

A sandwich assay utilizing the above starting materials and apparatus may be carried out as follows:

The avidin-coated waveguide is incubated with a solution of the biotin/R-phycoerythrin/anti-hCG antibody. The donor fluorophore-labelled antibody A is thus immobilised on the waveguide surface as a result of the high mutual affinity of avidin and biotin. The waveguide is then incubated with sample containing the antigen under assay (hCG) and a fixed amount of the allophycocyanin(acceptor fluorophore)-labelled antibody B. The waveguide is illuminated with radiation at a wavelength suitable for excitation of the donor fluorophore, i.e. around 485 nm for R-phycoerythrin. This illumination may be effected either after equilibrium has been attached or during the course of the attainment of equilibrium. The intensity of light emitted from the donor fluorophore is monitored, i.e. around 580 nm for R-phycoerythirin. As a result of formation of sandwich complex, the fluorescence intensity at 580 nm will decrease as a function of the concentration of hCG. Calibration may be effected by comparison of results with sample solutions containing zero or known amounts of hCG.

As mentioned above, competition-type assays may also be carried out by the method of the invention. The procedure in these cases is similar.

Competition assay for hCG

Antibody labelled with donor fluorophore (R-phycoerythrin) is immobilised at the waveguide surface by means of the avidin/biotin interaction, according to the method described above. Acceptor fluorophore (allophycocyanin) is conjugated to hCG molecules. The waveguide is then incubated with liquid sample containing hCG and a fixed, known amount of allophycocyanin/hCG conjugate. The donor fluorophores are excited by application of light of a suitable wavelength, and the attenuation of the fluorescence of the donor fluorophore is monitored. Fluorescence due to the donor fluorophore decrease with decreasing amount of hCG in the sample.

**Claims**

1.   A method of assay of a ligand in a sample which comprises
     (A) incubating, simultaneously or in any desired sequence,
          (a) the sample,
          (b) a reagent X labelled with a first energy acceptor,
          (c) a reagent Y immobilised directly or indirectly on the surface of an optical waveguide,
          (d) a second energy acceptor immobilised directly or indirectly (if desired via reagent Y) on the surface of the optical waveguide,
          (e) if desired, at least one further energy acceptor, which may be the same as or different from said first or second energy acceptors, immobilised directly or indirectly on or within the surface. of said optical waveguide, wherein one of the reagents X and Y comprises a specific binding partner to said ligand and the other of reagents X and Y comprises either a ligand analogue or a specific binding partner of said ligand and wherein one of the said first and second energy acceptors comprises a fluorophore (a "donor fluorophore") the electronic emission spectrum of which overlaps with the electronic absorption spectrum of the other of said first and second energy acceptors;
     (B) irradiating the said optical waveguide with electromagnetic radiation of a wavelength suitable for exciting said donor fluorophore; and
     (C) detecting whether and, if desired, the extent to which and/or rate at which, resonance energy transfer occurs, either directly or indirectly, between the first and second energy acceptors or between the second and first energy acceptors respectively: wherein either the irradiation of step (B) is by means of the evanescent field produced by electromagnetic radiation propagating along said waveguide and/or the detection step (C) utilises evanescent field coupling into the waveguide of the fluorescence of the first energy acceptor or the fluorescence, if any, of the second energy acceptor.

2. A method as claimed in claim 1 wherein the first energy acceptor comprises a donor fluorophore and the second energy acceptor comprises an acceptor dye or an acceptor fluorophore.

3. A method as claimed in claim 1 wherein the first energy acceptor comprises an acceptor dye or an acceptor fluorophore and the second energy acceptor comprises a donor fluorophore.

4. A method as claimed in any one of the preceding claims wherein an acceptor fluorophore is present as component (e).

5. A method as claimed in any one of the preceding claims wherein the irradiation step (B) utilizes the evanescent field associated with the electromagnetic radiation propagating along the optical waveguide.

6. A method as claimed in any one of the preceding claims wherein one of the energy acceptors comprises an acceptor fluorophore and the detection step (C) utilizes evanescent field coupling of the fluorescence emitted by the acceptor fluorophore into the waveguide.

7. A method as claimed in any one of the preceding claims wherein the said energy acceptors are selected from the group comprising phycobiliproteins, coumarins, fluorescein, rhodamines, lucifer yellows, erythrosin or derivatives thereof.

8. A method as claimed in claim 1 wherein the optical waveguide is made of glass or silica.

9. A kit for use in a method as claimed in claim 1 comprising
   (i) a reagent X labelled with a first energy acceptor; and
   (ii) an optical waveguide on the surface of which is immobilised (directly or indirectly) a reagent Y and on which is immobilised (directly, or indirectly, if desired via reagent Y) at least one further energy acceptor,
       the said reagents X and Y and the said energy acceptors being as defined in claim 1.

## Patentansprüche

1. Assayverfahren auf einen Liganden in einer Probe, umfassend
   (A) gleichzeitiges Inkubieren oder Inkubieren in irgendeiner gewünschten Reihenfolge
       (a) der Probe,
       (b) eines mit einem ersten Energieakzeptor markierten Reagenzes X,
       (c) eines direkt oder indirekt auf der Oberfläche eines optischen Hohlleiters immobilisierten Reagenzes Y,
       (d) eines direkt oder indirekt (gegebenenfalls über das Reagenz Y) auf der Oberfläche des optischen Hohlleiters immobilisierten zweiten Energieakzeptors,
       (e) gegebenenfalls mindestens eines weiteren Energieakzeptors, der dem ersten oder zweiten Energieakzeptor gleich oder verschieden davon sein kann, der direkt oder indirekt auf oder innerhalb der Oberfläche des optischen Hohlleiters immobilisiert ist, wobei eines der Reagenzien X und Y einen spezifischen Bindungspartner für den Liganden umfaßt, und das andere der Reagenzien X und Y entweder ein Ligandenanalogon oder einen spezifischen Bindungspartner des Liganden umfassen, und worin einer des ersten oder zweiten Energieakzeptors einen Fluorophor (ein "Donorfluorophor" ) umfaßt, dessen Elektronenemissionsspektrum mit dem Elektronenabsorptionsspektrum des anderen des ersten und zweiten Energieakzeptors überlappt,
   (B) elektromagnetisches Bestrahlen des optischen Hohlleiters mit einer zur Anregung des Donorfluorophors geeigneten Wellenlänge, und
   (C) Nachweisen, ob, und gegebenenfalls in welchem Ausmaß und/ oder mit welcher Geschwindigkeit der Resonanzenergietransfer entweder direkt oder indirekt zwischen dem ersten und zweiten Energieakzeptor bzw. zwischen dem zweiten und ersten Energieakzeptor eintritt, wobei entweder das Bestrahlen von Stufe (B) mittels des abklingenden Feldes, das durch elektromagnetische Strahlung, die sich entlang des Hohlleiters fortpflanzt, durchgeführt wird, und/oder bei der Nachweisstufe (C) die Kopplung des abklingenden Feldes in den Hohlleiter der Fluoreszenz des ersten Energieakzeptors oder - sofern vorhanden - der Fluoreszenz des zweiten Energieakzeptors verwendet wird.

2. Verfahren nach Anspruch 1, worin der erste Energieakzeptor einen Donorfluorophor umfaßt, und der zweite Energieakzeptor einen Akzeptorfarbstoff oder einen Akzeptorfluorophor umfaßt.

3. Verfahren nach Anspruch 1, worin der erste Energieakzeptor einen Akzeptorfarbstoff oder einen Akzeptorfluorophor umfaßt, und der zweite Energieakzeptor einen Donorfluorophor umfaßt.

4. Verfahren nach einem der vorausgehenden Ansprüche, worin ein Akzeptorfluorophor als Komponente (e) vorhanden ist.

5. Verfahren nach einem der vorausgehenden Ansprüche, worin man bei der Bestrahlung von Stufe (B) das abklingende Feld in Assoziation mit der elektromagnetischen Strahlung, die sich entlang des Hohlleiters fortpflanzt, verwendet.

6. Verfahren nach einem der vorausgehenden Ansprüche, worin einer der Energieakzeptoren einen Akzeptorfluorophor umfaßt, und man bei der Nachweisstufe (C) die Kopplung des abklingenden Feldes der von dem Akzeptorfluorophor emittierten Strahlung in den Hohlleiter verwendet.

7. Verfahren nach einem der vorausgehenden Ansprüche, worin man die Energieakzeptoren aus der Gruppe, umfassend Phycobiliproteine, Cumarine, Fluorescein, Rhodamine, Lucifer-Gelb, Erythrosin oder Derivate davon, auswählt.

8. Verfahren nach Anspruch 1, worin der optische Hohlleiter aus Glas oder Kieselsäure ist.

9. Kit zur Verwendung bei einem Verfahren nach Anspruch 1, umfassend
(i) ein mit einem ersten Energieakzeptor markiertes Reagenz X,
(ii) einen optischen Hohlleiter, auf dessen Oberfläche (direkt oder indirekt) ein Reagenz Y immobilisiert ist, und auf dem (direkt oder indirekt, gegebenenfalls über das Reagenz Y) mindestens ein weiterer Energieakzeptor immobilisiert ist, wobei die Reagenzien X und Y und die Energieakzeptoren wie in Anspruch 1 definiert sind.


## Revendications

1. Procédé d'analyse d'un ligand dans un échantillon, qui comprend :
(A) l'incubation, simultanément ou dans toute séquence désirée, de
(a) l'échantillon
(b) un réactif X marqué avec un premier accepteur d'énergie,
(c) un réactif Y immobilisé directement ou indirectement sur la surface d'un guide d'ondes optiques,
(d) un deuxième accepteur d'énergie immobilisé directement ou indirectement (si on le désire, par l'intermédiaire du réactif Y) sur la surface du guide d'ondes optiques,
(e) si on le désire, au moins un autre accepteur d'énergie, qui peut être le même que lesdits premier et deuxième accepteurs d'énergie ou différent de ceux-ci, immobilisé directement ou indirectement sur ou dans la surface dudit guide d'ondes optiques, un des réactifs X et Y comprenant un partenaire de liaison spécifique audit ligand et l'autre des réactifs X et Y comprenant un ligand analogue ou un partenaire de liaison spécifique dudit ligande, et un desdits premier et deuxième accepteurs d'énergie comprenant un fluorophore (un "fluorophore donneur") dont le spectre d'émission électronique chevauche le spectre d'absorption électronique de l'autre desdits premier et deuxième accepteurs d'énergie ;
(B) l'irradiation dudit guide d'ondes optiques avec un rayonnement électromagnétique d'une longueur d'onde appropriée pour l'excitation dudit fluorophore donneur; et
(C) la détection d'un transfert d'énergie de résonance et, si on le désire, de l'étendue à laquelle et/ou de la vitesse à laquelle ce transfert a lieu, directement ou indirectement, entre les premier et deuxième accepteurs d'énergie ou entre les deuxième et premier accepteurs d'énergie respectivement ;
dans lequel l'irradiation de l'étape (B) est effectuée au moyen du champ évanescent produit par le rayonnement électromagnétique qui se propage le long dudit guide d'ondes, et/ou l'étape de détection (C) utilise un couplage de champ évanescent dans le guide d'ondes de la fluorescence du premier accepteur d'énergie ou de la fluorescence, si elle existe, du deuxième accepteur d'énergie.

2. Procédé suivant la revendication 1, dans lequel le premier accepteur d'énergie comprend un fluorophore donneur et le deuxième accepteur d'énergie comprend un colorant accepteur ou un fluorophore accepteur.

3. Procédé suivant la revendication 1, dans lequel le premier accepteur d'énergie comprend un colorant accepteur ou un fluorophore accepteur et le deuxième accepteur d'énergie comprend un fluorophore donneur.

4. Procédé suivant une quelconque des revendications précédentes, dans lequel un fluorophore accepteur est présent comme composant (e).

5. Procédé suivant une quelconque des revendications précédentes, dans lequel l'étape d'irradiation (B) utilise le champ évanescent associé au rayonnement électromagnétique qui se propage le long du guide d'ondes optiques.

6. Procédé suivant une quelconque des revendications précédentes, dans lequel un des accepteurs d'énergie comprend un fluorophore accepteur et l'étape de détection (C) utilise le couplage de champ évanescent de la fluorescence émise par le fluorophore accepteur dans le guide d'ondes.

7. Procédé suivant une quelconque des revendications précédentes, dans lequel lesdits accepteurs d'énergie sont choisis dans le groupe comprenant phycobiliprotéines , coumarines,fluorescéine, rhodamines, jaunes lucifères,érythrosine ou leurs dérivés.

8. Procédé suivant la revendication 1, dans lequel le guide d'ondes optiques est en verre ou en silice.

9. Nécessaire utilisable dans un procédé suivant la revendication 1, comprenant :
(i) un réactif X marqué avec un premier accepteur d'énergie ; et
(ii) un guide d'ondes optiques sur la surface duquel est immobilisé (directement ou indirectement) un réactif Y et sur lequel est immobilisé (directement ou indirectement, si on le désire par l'intermédiaire du réactif Y) au moins un autre accepteur d'énergie ;
lesdits réactifs X et Y et lesdits accepteurs d'énergie étant comme défini dans la revendication 1.

FIG.1.

FIG.2.

FIG.3.

FIG.4.

FIG.5.

FIG.6.

FIG.7.

FIG.8.

FIG.9.

FIG.10.

FIG.11.

EP 0 359 807 B1